(19) **Europäisches Patentamt / European Patent Office / Office européen des brevets**

(11) **EP 1 339 411 B1**

(12) **EUROPEAN PATENT SPECIFICATION**

(45) Date of publication and mention
of the grant of the patent:
**04.07.2007 Bulletin 2007/27**

(51) Int Cl.:
*A61K 31/66* (2006.01)     *A61P 19/00* (2006.01)

(21) Application number: **01998352.7**

(22) Date of filing: **27.11.2001**

(86) International application number:
**PCT/EP2001/013836**

(87) International publication number:
**WO 2002/043738 (06.06.2002 Gazette 2002/23)**

(54) **USE OF ZOLEDRONIC ACID FOR PAIN TREATMENT**

VERWENDUNG VON ZOLEDRONSÄURE ZUR SCHMERZBEHANDLUNG

UTILISATION DE ACIDE ZOLEDRONIQUE DANS LE TRAITEMENT DE LA DOULEUR

(84) Designated Contracting States:
**AT BE CH CY DE DK ES FI FR GB GR IE IT LI LU MC NL PT SE TR**
Designated Extension States:
**SI**

(30) Priority: **29.11.2000 GB 0029111**

(43) Date of publication of application:
**03.09.2003 Bulletin 2003/36**

(73) Proprietors:
• **Novartis AG**
**4056 Basel (CH)**
Designated Contracting States:
**BE CH CY DE DK ES FI FR GB GR IE IT LI LU MC NL PT SE TR**
• **Novartis Pharma GmbH**
**1230 Wien (AT)**
Designated Contracting States:
**AT**

(72) Inventors:
• **FOX, Alyson,**
**The Novartis Inst. for Med. Sciences**
**London, WC1E 6BN (GB)**
• **GREEN, Jonathan**
**CH-4144 Arlesheim (CH)**
• **O'REILLY, Terence**
**CH-4057 Basel (CH)**
• **URBAN, Laszlo,**
**The Novartis Inst. for Medical Sci.**
**London, WC1E 6BN (GB)**
• **WALKER, Katharine**
**Princeton, NJ 08540 (US)**

(74) Representative: **Gros, Florent**
**Novartis AG**
**4002 Basel (CH)**

(56) References cited:
**WO-A-00/28954          WO-A-95/30421**

• **BERENSON, J. R. ET AL.: "EFFICACY OF PAMIDRONATE IN REDUCING SKELETAL EVENTS IN PATIENS WITH ADVANCED MULTIPLE MYELOMA" NEW ENGLAND JOURNAL OF MEDICINE, vol. 334, no. 8, 22 February 1996 (1996-02-22), pages 488-493, XP008002467 cited in the application**
• **HORTOBAGYI, G. N. ET AL.: "EFFICACY OF PAMIDRONATE IN REDUCING SKELETAL COMPLICATIONS IN PATIENTS WITH BREAST CANCER AND LYTIC BONE METASTASES" NEW ENGLAND JOURNAL OF MEDICINE, vol. 335, no. 24, 12 December 1996 (1996-12-12), pages 1785-1791, XP008002503 cited in the application**
• **MCCLOSKEY, E. V. ET AL.: "A RAMDOMIZED TRIAL OF THE EFFECT OF CLODRONATE ON SKELETAL MORBIDITY IN MULTIPLE MYELOMA" BRITISH JOURNAL OF HAEMATOLOGY, vol. 100, 1998, pages 317-325, XP002196185 cited in the application**
• **FULFARO, F. ET AL.: "The role of bisphosphonates in the treatment of painful metastatic bone disease: a review of trials III trials" PAIN, vol. 78, 27 June 1998 (1998-06-27), pages 157-169, XP002211230**
• **REGINSTER, J.-Y. ET AL.: "Efficacy and Safety of Drugs for Paget's Disease of Bone" BONE, vol. 17, no. 5 Supplement, November 1995 (1995-11), pages 485S-488S, XP002211231**

EP 1 339 411 B1

- **ROGERS M J ET AL: "OVERVIEW OF BISPHOSPHONATES" CANCER, AMERICAN CANCER SOCIETY, PHILADELPHIA, PA, US, vol. 80, no. 8, SUPPL, 1997, pages 1652-1660, XP001038422 ISSN: 0008-543X**

**Description**

This invention relates to the use of zoledronic acid for the preparation of a medicament for the treatment of pain wherein the medicament is to be administered once every six months.

Bisphosphonates are widely used to inhibit osteoclast activity in a variety of both benign and malignant diseases which involve excessive or inappropriate bone resorption. These pyrophosphate analogs not only reduce the occurrence of skeletal related events but they also provide patients with clinical benefit and improve survival. Bisphosphonates are able to prevent bone resorption in vivo; the therapeutic efficacy of bisphosphonates has been demonstrated in the treatment of osteoporosis, osteopenia, Paget's disease of bone, tumour-induced hypercalcemia (TIH) and, more recently, bone metastases (BM) and multiple myeloma (MM) (for review see Fleisch H 1997 Bisphosphonates clinical. In Bisphosphonates in Bone Disease. From the Laboratory to the Patient. Eds: The Parthenon Publishing Group, New York/London pp 68-163). The mechanisms by which bisphosphonates inhibit bone resorption are still not completely understood and seem to vary according to the bisphosphonates studied. Bisphosphonates have been shown to bind strongly to the hydroxyapatite crystals of bone, to reduce bone turn-over and resorption, to decrease the levels of hydroxyproline or alkaline phosphatase in the blood, and in addition to inhibit the formation, recruitment, activation and the activity of osteoclasts. Recently farnesyl diphosphate synthase, an enzyme of the mevalonate pathway of cholesterol biosynthesis, has been identified as the molecular target of nitrogen-containing bisphosphonates (reviewed in Rogers MJ, Gordon S, Benford HL, Coxon FP, Luckman SP, Monkkonen J, Frith JC. 2000. Cellular and molecular mechanisms of action of bisphosphonates. Cancer 88(suppl):2961-2978)

Fulfaro, F. et al, Pain, vol 78, 27. June 1998, pages 157-169 describes that the treatment of metastatic bone diseases is accompanied by severe pain and that zoledronate has an analgesic effect.

Bone pain resulting from structural damage, periosteal irritation, and nerve entrapment is the most common complication of both benign and metastatic bone disease, and presents a significant problem in both hospital and community practice (Coleman, 1997, Cancer 80; 1588-1594).

MM is a plasma-cell malignancy characterized by the proliferation and the accumulation of malignant plasma cells within the bone marrow. The main clinical consequences are lytic bone lesions associated with pathologic fractures and bone pain. These lesions result from an excessive bone resorption, frequently leading to hypercalcemia. Bisphosphonates have been introduced for the long-term treatment of MM in combination with conventional chemotherapy. It has been shown recently that bisphosphonates such as clodronate and pamidronate can reduce the occurrence of skeletal related events such as lytic bone lesions and pathologic fractures and can relieve associated bone pain and improve the quality of life of patients (Laktinen et al. Lancet 1992, 340, 1049-1052; McCloskey et aL B.J. Haematol, 1998, 100, 317-325; and Berenson et al. N. Eng. J. Med. 1996, Vol. 334, No. 8, 488-493). Similar effects have been reported in breast cancer patients treated with bisphosphonates (Hortobagyi GN, Theriault RL, Porter L, Blayney D, Lipton A, Sinoff C, Wheeler H, Simeone JF, Seaman J, Knight RD. Efficacy of pamidronate in reducing skeletal complications in patients with breast cancer and lytic bone metastases. Protocol 19 Aredia Breast Cancer Study Group. N Engl J Med. 1996;335:1785-91; Kanis JA, Powles T, Paterson AHG, McCloskey EV, Ashley S. Clodronate decreases the frequency of skeletal metastases in women with breast cancer. Bone 1996; 19: 663-7.)

It has now been found surprisingly that zoledronic acid exert profound and apparently direct palliative effects on pain in in vivo animal models. For example, zoledronic acid has been found to reverse mechanical hyperalgesia in rat models of chronic inflammatory and neuropathic pain, with a fast onset of action and efficacy of up to about 100%. Additionally zoledronic acid has been found to reduce mechanical allodynia and reduce hind limb sparing in a rat model of bone cancer pain. These results indicate that zoledronic acid may have direct, fast acting, anti-nociceptive and anti-allodynic activity on pain.

Accordingly the present invention provides the use according to claim 1.

The present invention is particularly applicable to the palliative treatment of pain, i.e. the direct relief of pain in addition to the relief of pain as the result of amelioration of the underlying disease or medical condition, which is the cause of the pain. Thus, advantageously the invention provides methods and uses for the direct analgesic or acute treatment of pain.

Preferably the invention is used for the direct treatment of pain in diseases and medical conditions in which bisphosphonates are used to inhibit osteoclast activity. For example, the invention may be used for direct treatment of pain in diseases and conditions which involve excessive or inappropriate bone loss e.g. as the result of inappropriate osteoclast activity. Examples of such diseases and conditions include benign diseases and conditions such as osteoporosis of various genesis, Pagets disease, osteoarthritis, RA, periodontal disease; and especially malignant diseases such as MM and TIH and BM associated with various cancers, e.g. cancer of the breast, prostate, lung, kidney, ovary, or osteosarcoma. Generally the invention may be used to treat pain in other circumstances where bisphosphonates are used and pain is encountered, e.g. when bisphosphonates are use in bone fracture healing, osteonecrosis or treatment of prosthesis loosening.

Thus in the present description the terms "treatment" or "treat" refer to both prophylactic or preventative treatment as well as curative or palliative treatment of pain, in particular anti-nociceptive and anti-allodynic treatment of pain, especially treatment of bone pain.

Pharmacologically acceptable salts of zoledronic acid are preferably salts with bases, conveniently metal salts derived from groups Ia, Ib, IIa and IIb of the Periodic Table of the Elements, including alkali metal salts, e.g. potassium and especially sodium salts, or alkaline earth metal salts, preferably calcium or magnesium salts, and also ammonium salts with ammonia or organic amines.

Especially preferred pharmaceutically acceptable salts are those where one, two, three or four, in particular one or two, of the acidic hydrogens of the bisphosphonic acid are replaced by a pharmaceutically acceptable cation, in particular sodium, potassium or ammonium, in first instance sodium.

A very preferred group of pharmaceutically acceptable salts is characterized by having one acidic hydrogen and one pharmaceutically acceptable cation, especially sodium, in each of the phosphonic acid groups.

The zoledronic acid (hereinafter referred to as the Agent of the Invention) may be used in the form of an isomer or of a mixture of isomers where appropriate, typically as optical isomers such as enantiomers or diastereoisomers or geometric isomers, typically cis-trans isomers. The optical isomers are obtained in the form of the pure antipodes and/or as racemates.

The Agent of the Invention can also be used in the form of its hydrates or includes other solvents used for its crystallisation.

The Agent of the Invention is preferably used in the form of pharmaceutical compositions that contain a therapeutically effective amount of active ingredient optionally together with or in admixture with inorganic or organic, solid or liquid, pharmaceutically acceptable carriers which are suitable for administration.

The pharmaceutical compositions may be, for example, compositions for enteral, such as oral, rectal, aerosol inhalation or nasal administration, compositions for parenteral, such as intravenous or subcutaneous administration, or compositions for transdermal administration (e.g. passive or iontophoretic).

Preferably, the pharmaceutical compositions are adapted to oral or parenteral (especially intravenous, intra-arterial or transdermal) administration. Intravenous and oral, first and foremost intravenous, administration is considered to be of particular importance. Preferably the active ingredient is in the form of a parenteral, most preferably an intravenous form.

The particular mode of administration and the dosage may be selected by the attending physician taking into account the particulars of the patient, especially age, weight, life style, activity level, hormonal status (e.g. post-menopausal) and bone mineral density as appropriate. Most preferably, however, the bisphosphonate is administered intravenously.

The dosage of the Agent of the Invention may depend on various factors, such as effectiveness and duration of action of the active ingredient, mode of administration, warm-blooded species, and/or sex, age, weight and individual condition of the warm-blooded animal.

Normally the dosage is such that a single dose of the zoledronic acid active ingredient from 0.002 - 20.0 mg/kg, especially 0.01 -10.0 mg/kg, is administered to a warm-blooded animal weighing approximately 75kg. If desired, this dose may also be taken in several, optionally equal, partial doses.

"mg/kg" means mg drug per kg body weight of the mammal - including man - to be treated.

The dose mentioned above administered as a single dose may be repeated once every six months or once a year.

Preferably, the zoledronic acidis administered in doses which are in the same order of magnitude as those used in the treatment of the diseases classically treated with zoledronic acid such as Paget's disease, tumour-induced hypercalcemia or osteoporosis. In other words, preferably zoledronic acid derivatives are administered in doses which would likewise be therapeutically effective in the treatment of Paget's disease, tumour-induced hypercalcaemia or osteoporosis, i.e. preferably they are administered in doses which would likewise effectively inhibit bone resorption. For example, for zoledronic acid and salts thereof, doses are in the range from about 0.5 to about 20mg, preferably from about 1 to about 10 mg, may be used for treatment of human patients.

Formulations in single dose unit form contain preferably from about 1% to about 90% active ingredient. Single dose unit forms such as capsules, tablets or dragées contain e.g. from about 1mg to about 500mg of the active ingredient.

Pharmaceutical preparations for enteral and parenteral administration are, for example, those in dosage unit forms, such as dragées, tablets or capsules and also ampoules. They are prepared in a manner known *per se,* for example by means of conventional mixing, granulating, confecting, dissolving or lyophilising processes.

For example, pharmaceutical preparations for oral administration can be obtained by combining the active ingredient with solid carriers, where appropriate granulating a resulting mixture, and processing the mixture or granulate, if desired or necessary after the addition of suitable adjuncts, into tablets or dragée cores. Suitable carriers are especially fillers, such as sugars, for example lactose, saccharose, mannitol or sorbitol, cellulose preparations and/or calcium phosphates, for example tricalcium phosphate or calcium hydrogen phosphate, and also binders, such as starch pastes, using, for example, corn, wheat, rice or potato starch, gelatin, tragacanth, methylcellulose and/or polyvinylpyrrolidone and, if desired, disintegrators, such as the above-mentioned starches, also carboxymethyl starch, crosslinked polyvinylpyrrolidone, agar or alginic acid or a salt thereof, such as sodium alginate. Adjuncts are especially flow-regulating agents and lubricants, for example silicic acid, talc, stearic acid or salts thereof, such as magnesium or calcium stearate, and/or polyethylene glycol. Dragee cores are provided with suitable coatings that may be resistant to gastric juices, there being used, inter alia. concentrated sugar solutions that optionally contain gum arabic, talc, polyvinylpyrrolidone, polyethylene glycol and/or titanium dioxide, or lacquer solutions in suitable organic solvents or solvent mixtures or, to produce coatings that are resistant to gastric juices, solutions of suitable cellulose preparations, such as acetylcellulose phthalate or hydroxypropylmethylcellulose phthalate. Colouring substances or pigments may be added to the tablets or dragee coatings, for example for the purpose of identification or to indicate different doses of active ingredient.

Other orally administrable pharmaceutical preparations are dry-filled capsules made of gelatin, and also soft, sealed capsules made of gelatin and a plasticiser, such as glycerol or sorbitoL The dry-filled capsules may contain the active ingredient in the form of a granulate, for example in admixture with fillers, such as lactose, binders, such as starches, and/or glidants, such as talc or magnesium stearate, and, where appropriate, stabilisers. In soft-capsules the active ingredient is preferably dissolved or suspended in suitable liquids, such as fatty oils, paraffin oil or liquid polyethylene glycols, it being possible also for stabilisers to be added.

Parenteral formulations are especially injectable fluids that are effective in various manners, such as intra-arterially, intramuscularly, intraperitoneally, intranasally, intradermally, subcutaneously or preferably intravenously. Such fluids are preferably isotonic aqueous solutions or suspensions which can be prepared before use, for example from lyophilised preparations which contain the active ingredient alone or together with a pharmaceutically acceptable carrier. The pharmaceutical preparations may be sterilised and/or contain adjuncts, for example preservatives, stabilisers, wetting agents and/or emulsifiers, solubilisers, salts for regulating the osmotic pressure and/or buffers.

Suitable formulations for transdermal application include an effective amount of the active ingredient with carrier. Advantageous carriers include absorbable pharmacologically acceptable solvents to assist passage through the skin of the host. Characteristically, transdermal devices are in the form of a bandage comprising a backing member, a reservoir containing the compound optionally with carriers, optionally a rate controlling barrier to deliver the active ingredient of the skin of the host at a controlled and predetermined rate over a prolonged period of time, and means to secure the device to the skin.

The following Examples illustrate the invention described hereinbefore.

Exampe 1 Monolith adhesive transdermal system, containing zoledronic acid as active ingredient, 1-hydroxy-2-(imidazol-1-yl)-ethane-1,1-diphosphonic acid:

Composition:

| | |
|---|---|
| polyisobutylene (PIB) 300 (Oppanol B1, BASF) | 5.0 g |
| PIB 35000 (Oppanol B 10, BASF) | 3.0 g |
| PIB 1200000 (Oppanol B100, BASF) | 9.0 g |
| hydrogenated hydrocarbon resin (Escorez 5320, Exxon) | 43.0 g |
| 1-dodecylazacycloheptan-2-one (Azone, Nelson Res., Irvine/CA) | 20.0 g |
| active ingredient | 20.0g |
| Total | 100.0 g |

Preparation:

The above components are together dissolved in 150 g of special boiling point petroleum fraction 100-125 by rolling on a roller gear bed. The solution is applied to a polyester film (Hostaphan, Kalle) by means of a spreading device using a 300mm doctor blade, giving a coating of about 75 $g/m^2$. After drying (15 minutes at 60°C), a silicone-treated polyester film (thickness 75 mm, Laufenberg) is applied as the peel-off film. The finished systems are punched out in sizes in the wanted form of from 5 to 30cm$^2$ using a punching tool. The complete systems are sealed individually in sachets of aluminised paper.

Example 2 : Vial containing 1.0 mg dry, lyophilized 1-hydroxy-2-(imidazol-1-yl)ethane-1,1-diphosphonic acid (mixed sodium salts thereof). After dilution with 1 ml of water, a solution (concentration 1 mg/ml) for i.v. infusion is obtained.

Composition:

| | | |
|---|---|---|
| active ingredient (free diphosphonic acid) | | 1.0 mg |
| mannitol | | 46.0 mg |
| Trisodium citrate x 2 $H_2O$ | ca. | 3.0 mg |
| water | | 1 ml |
| water for injection | | 1 ml. |

In 1 ml of water, the active ingredient is titrated with trisodium citrate x 2 $H_2O$ to pH 6.0. Then, the mannitol is added and the solution is lyophilized and the lyophilisate filled into a vial. a solution

Example 3 The Effect of Zoledronate in Rat Models of Inflammatory and Neuropathic Pain

**Methods**

*Inflammatory hyperalgesia*

Mechanical hyperalgesia was examined in a rat model of inflammatory pain. Paw withdrawal thresholds to an increasing pressure stimulus were measured by the Randal-Sellito technique using an analgesymeter (Ugo Basile, Milan), in naïve animals prior to an intraplantar injection of complete Freund's complete adjuvant (FCA) into the left hind paw. 24 h later paw withdrawal thresholds were measured again prior to (predose) and then from 10 min to 6 h following drug or vehicle administration. Reversal of hyperalgesia in the ipsilateral paw was calculated according to the formula:

$$\% \; reversal = \frac{postdose \; threshold - predose \; threshold}{na\ddot{i}ve \; threshold - predose \; threshold} \; X \; 100$$

*Neuropathic hyperalgesia*

Mechanical hyperalgesia was examined in a rat model of neuropathic pain induced by partial ligation of the left sciatic nerve. Approximately 14 days following surgery mechanical withdrawal thresholds of both the ligated (ipsilateral) and non-ligated (contralateral) paw were measured prior to (predose) and then from 10 min to 6 h following drug or vehicle administration. Reversal of hyperalgesia at each time point was calculated according to the formula:

$$\% \; reversal = \frac{ipsilateral \; threshold \; postdose - ipsilateral \; threshold \; predose}{contralateral \; threshold \; predose - ipsilateral \; threshold \; predose} \; X \; 100$$

All experiments were carried out using groups of 6 animals. Stock concentrations of drugs were dissolved in distilled water and subsequent dilutions were made in 0.9% saline for subcutaneous administration in a volume of 4 mlkg[-1]. All drugs were made up in plastic vials and kept in the dark.

Statistical analysis was carried out on withdrawal threshold readings (g) using ANOVA with repeated measures followed by Tukey's HSD test. Efficacy refers to the maximal reversal of hyperalgesia observed at the doses used.

**Results**

1. In a model of inflammatory hyperalgesia induced by unilateral hindpaw injection of complete Freund's adjuvant Zoledronate (0.003 - 0.1 mgkg$^{-1}$ s.c.) produced a dose-dependant reversal of mechanical hyperalgesia. The effect was rapid in onset, with a maximal reversal of 100 % within 30 min, and of short duration with no significant activity 3 h following administration. Some contralateral activity was observed at the highest dose.

2. In a model of chronic neuropathic pain induced by unilateral partial sciatic nerve ligation Zoledronate (0.003 - 0.1 mgkg$^{-1}$ s.c.) produced a moderate 40 % reversal of mechanical hyperalgesia which was maximal within 30 min of administration. However, there was also a significant reduction in contralateral paw withdrawal thresholds at the highest dose.

3. These data show that Zoledronate reverses mechanical hyperalgesia in models of chronic inflammatory and neuropathic pain in the rat.

**Claims**

1. Use of zoledronic acid or a pharmaceutically acceptable salt thereof, or any hydrate thereof, in the preparation of a medicament for the treatment of pain wherein the medicament is to be administered once every six months.

2. Use of zoledronic acid or a pharmaceutically acceptable salt thereof, or any hydrate thereof, in the preparation of a medicament for the treatment of pain wherein the medicament is to be administered once yearly.

3. Use according to claim 1 or claim 2 wherein the medicament is for the treatment of bone pain.

4. Use according to any one of the preceding claims wherein the medicament is for the treatment of pain associated with osteopenaa, Paget's disease, osteoporosis, rheumatoid arthritis or osteoarthritis.

5. Use according to any one of the preceding claims wherein the medicament is adapted for parenteral administration.

**Patentansprüche**

1. Verwendung von Zoledronsäure oder einem pharmazeutisch akzeptablen Salz hiervon oder irgendeinem Hydrat hiervon zur Herstellung eines Arzneimittels für die Behandlung von Schmerz, worin das Arzneimittel einmal alle sechs Monate zu verabreichen ist.

2. Verwendung von Zoledronsäure oder einem pharmazeutisch akzeptablen Salz hiervon oder irgendeinem Hydrat hiervon zur Herstellung eines Arzneimittels für die Behandlung von Schmerz, worin das Arzneimittel einmal jährlich zu verabreichen ist.

3. Verfahren nach Anspruch 1 oder 2, worin das Arzneimittel für die Behandlung von Knochenschmerz ist.

4. Verwendung nach einem der vorhergehenden Ansprüche, worin das Arzneimittel für die Behandlung von Knochenschmerz ist, der assoziiert ist mit Osteopenie, Paget-Krankheit, Osteoporose, rheumatoider Arthritis oder Osteoarthritis.

5. Verwendung nach einem der vorhergehenden Ansprüche, worin das Arzneimittel für eine parenterale Verabreichung angepasst ist.

**Revendications**

1. Utilisation de l'acide zolédronique ou d'un de ses sels pharmaceutiquement acceptables, ou de n'importe lequel de ses hydrates, dans la préparation d'un médicament destiné au traitement de la douleur, **caractérisée en ce que** le médicament doit être administré une fois tous les six mois.

2. Utilisation de l'acide zolédronique ou d'un de ses sels pharmaceutiquement acceptables, ou de n'importe lequel de ses hydrates, dans la préparation d'un médicament destiné au traitement de la douleur, **caractérisée en ce que** le médicament doit être administré une fois par an.

**3.** Utilisation selon la revendication 1 ou 2, **caractérisée en ce que** le médicament est destiné au traitement de la douleur osseuse.

**4.** Utilisation selon l'une quelconque des revendications précédentes, **caractérisée en ce que** le médicament est destiné au traitement de la douleur associée à l'ostéopénie, à la maladie de Paget, à l'ostéoporose, à l'arthrite rhumatoïde ou à l'ostéoarthrite.

**5.** Utilisation selon l'une quelconque des revendications précédentes, **caractérisée en ce que** le médicament est adapté à une administration par voie parentérale.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- Bisphosphonates clinical. **FLEISCH H.** Bisphosphonates in Bone Disease. The Parthenon Publishing Group, 1997, 68-163
- **ROGERS MJ ; GORDON S ; BENFORD HL ; COXON FP ; LUCKMAN SP ; MONKKONEN J ; FRITH JC.** Cellular and molecular mechanisms of action of bisphosphonates. *Cancer,* 2000, vol. 88, 2961-2978
- **FULFARO, F. et al.** *Pain,* 27 June 1998, vol. 78, 157-169
- **COLEMAN.** *Cancer,* 1997, vol. 80, 1588-1594
- **LAKTINEN et al.** *Lancet,* 1992, vol. 340, 1049-1052, Lancet
- **MCCLOSKEY et al.** *B.J. Haematol,* 1998, vol. 100, 317-325, B.J. Haematol
- **BERENSON et al.** *N. Eng. J. Med,* 1996, vol. 334 (8), 488-493
- **HORTOBAGYI GN ; THERIAULT RL ; PORTER L ; BLAYNEY D ; LIPTON A ; SINOFF C ; WHEELER H ; SIMEONE JF ; SEAMAN J ; KNIGHT RD.** Efficacy of pamidronate in reducing skeletal complications in patients with breast cancer and lytic bone metastases. *Protocol 19 Aredia Breast Cancer Study Group. N Engl J Med.,* 1996, vol. 335, 1785-91, Protocol 19 Aredia Breast Cancer Study Group. N Engl J Med.
- **KANIS JA ; POWLES T ; PATERSON AHG ; MCCLOSKEY EV ; ASHLEY S.** Clodronate decreases the frequency of skeletal metastases in women with breast cancer. *Bone,* 1996, vol. 19, 663-7, Bone